(19) Europäisches Patentamt
European Patent Office
Office européen des brevets

(11) Numéro de publication: **0 214 055 B1**

# (12) FASCICULE DE BREVET EUROPEEN

(45) Date de publication du fascicule du brevet: **27.12.89**

(51) Int. Cl.⁴: **A61K 31/685**, C11D 1/88

(21) Numéro de dépôt: **86401896.5**

(22) Date de dépôt: **29.08.86**

(54) **Nouveaux surfactants artificiels, leur préparation et les compositions pharmaceutiques qui les contiennent.**

(30) Priorité: **30.08.85 FR 8512950**

(43) Date de publication de la demande:
**11.03.87 Bulletin 87/11**

(45) Mention de la délivrance du brevet:
**27.12.89 Bulletin 89/52**

(84) Etats contractants désignés:
**AT BE CH DE FR GB IT LI LU NL SE**

(56) Documents cités:
**EP-A- 0 050 793**
**EP-A- 0 110 498**
**DE-A- 2 900 300**

(73) Titulaire: **ADIR ET COMPAGNIE, 22, rue Garnier, F-92200 Neuilly-sur-Seine(FR)**

(72) Inventeur: **Bonte, Frédéric, 127 bis rue du Vieux Pont de Sèvres, F-92100 Boulogne(FR)**
Inventeur: **Dehan, Michel, 44 avenue Gabriel Péri, F-91370 Verrières le Buisson(FR)**
Inventeur: **Le Ridant, Alain, 52 bd. Général Leclerc, F-92200 Neuilly sur Seine(FR)**
Inventeur: **Puisieux, Francis, 66 rue de Strasbourg, F-94700 Maisons Alfort(FR)**
Inventeur: **Taupin, Christiane Colette, 6 allée François Villon, F-91400 Orsay(FR)**

**EP 0 214 055 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention se rapporte à de nouveaux surfactants articifiels pulmonaires, à leur procédé de préparation et aux compositions pharmaceutiques qui les contiennent.

On appelle surfactant pulmonaire l'ensemble des substances tensio-actives qui diminuent la tension superficielle de la surface de séparation gaz-liquide alvéolaire. Le surfactant pulmonaire, qui s'étale en un film très fin (mono-couche) à la surface des millions de petites alvéoles pulmonaires,joue un rôle physiologique très important. Il permet d'abaisser la tension superficielle intra-alvéolaire lors de l'expiration, rendant ainsi possible le maintien d'un volume résiduel de gaz, nécessaire à la permanence des échanges d'oxygène et de dioxyde de carbone entre le capillaire pulmonaire et le gaz alvéolaire. Une déficience en surfactant endogène provoque des troubles de la fonction respiratoire.

Il est connu que ces troubles chez les enfants nés prématurément sont responsables de la maladie des membranes hyalines. D'autre part, des déficiences importantes en surfactant endogène sont aussi rencontrées chez les adultes ayant des infections pulmonaires, chez des intoxiqués par les gaz et chez certains grands brûlés.

Pour le traitement des déficiences en surfactant endogène et dans un but de remplacer le surfactant pulmonaire défaillant, on a employé récemment des surfactants exogènes, d'origine naturelle ou des compositions artificielles contenant des matières tensio-actives. Les surfactants exogènes sont caractérisés par un certain nombre de propriétés physicochimiques essentielles pour leurs utilisations dans le but recherché. En effet, ils doivent être actifs à 37°C et pouvoir s'étaler rapidement à l'interface alvéolaire sous forme d'un film monomoléculaire. Cette monocouche de surfactant comprimée à l'interface air-eau doit permettre l'obtention d'une basse tension de surface (entre 0 et 9 $mN \cdot m^{-1}$) pour éviter le collapsus alvéolaire et ceci pendant un temps suffisamment long au cours des cycles respiratoires. De plus, les surfactants exogènes doivent assurer la permencence des échanges gazeux alvéolocapillaires, être non toxiques, et bien tolérés lors de leurs utilisations en thérapeutique.

Les produits tensio-actifs pulmonaires extraits du poumon des mammifères ont les propriétés indiquées ci-dessus, mais ils contiennent inévitablement une quantité considérable de protéines dites étrangères et sont contaminés généralement par beaucoup de micro-organismes. On ne peut donc utiliser ces produits qu'après une purification souvent très longue et onéreuse.

Plusieurs procédés sont actuellement connus (Brevet Français n°80.11680, Demande Japonaise N° 58 164 513, Demande Japonaise N°58,183,620, Demande Européenne N° 119 056, Demande Japonaise N° 58 045 299) mais leur application est peu intéressante à cause de l'approvisionnement difficile et la variabilité des matières premières d'origine animale.

L'expérience a montré que l'utilisation en thérapeutique des surfactants artificiels est possible à condition qu'ils possèdent un certain nombre de caractéristiques physiques et qu'ils aient une composition chimique proche du surfactant naturel.

La composition biochimique du surfactant humain n'est pas connue avec certitude du fait de sa complexité et de la diversité des méthodes utilisées, mais on admet généralement qu'il est constitué de 85 % de phospholipides, 13 % de protéines et 2 % de substances diverses. La fraction lipidique responsable de la quasi-totalité du pouvoir tensio-actif est constituée principalement de phospholipides saturés. Le composé majeur est la dipalmitoylphosphatidylcholine qui est connue pour abaisser la tension de surface alvéolaire en dessous d'un $mN \cdot m^{-1}$ sous compression dynamique. Plusieurs mélanges artificiels contenant des glucides, des aminoacides, des acides gras ou des alcools de grand poids moléculaire et ayant comme constituant principal la dipalmitoylphophatidylcholine sont déjà connus (Demandes Japonaises N° 58 183 621, 58 135 813, Brevet Américain N° 4 312 860, Demande Allemande N° 29 00 300)mais ce sont surtout des mélanges binaires de phospholipides contenant aussi du phosphatidylglycérol qui ont été décrits et utilisés préférentiellement en thérapeutique. (C.J. Morley et all. The Lancet, 1981, 1 64-68, Demande Allemande N° 32 29 179, Demande Européenne 110 498). En effet, pendant longtemps le phosphatidylglycérol a été considéré comme l'un des constituants indispensables d'un surfactant artificiel.

Or, jusqu'à présent, les essais in vitro ont démontré qu'aucun surfactant artificiel contenant de la dipalmitoylphosphatidylcholine et du phosphatidylglycérol ne possède des propriétés de surface identiques à celles définies par l'étude du surfactant naturel. Ces résultats laissaient présager une activité moyenne in vivo ce qui a d'ailleurs été confirmé après leurs essais en clinique. D'autre part, l'utilisation du phosphatidylglycérol pour la préparation des surfactants artificiels présente plusieurs inconvénients. Il est d'approvisionnement onéreux et délicat car son instabilité implique sa conservation dans un solvant organique à une température très inférieure de l'ambiante. De plus, comme il est obtenu par voie enzymatique à partir de la lécithine d'oeuf, ces lots ont des caractéristiques physicochimiques très variables.

La demanderesse a maintenant découvert un ensemble de surfactants artificiels, mis en oeuvre facilement avec des matières premières accessibles industriellement, et ayant toutes les caractéristiques physiques du surfactant naturel humain.

L'invention a plus précisément pour objet des surfactants artificiels, caractérisés en ce qu'ils contiennent des mélanges ternaires de dipalmitoylphosphatidylcholine, de distéaroylphosphatidylcholine et de lécithine de soja et en ce qu'ils sont constitués par:

1/ 10 à 70 % en moles de dipalmitoylphosphatidylcholine,
2/ 10 à 70 % en moles de distéaroylphosphatidylcholine,
3/ 10 à 30 % en moles de lécithine de soja,

la somme des constituants indiquée sous 1 à 3 devant, dans chaque cas, être égale à 100 % en moles.

En vue de leur emploi pour compenser le déficit en matière tensio-active pulmonaire endogène, les surfactants artificiels de l'invention, sont présentés sous diverses formes pharmaceutiques stériles, convenant pour l'administration directe dans la trachée-artère.

A titre d'exemple non limitatif, on pourra citer la forme liposome. La concentration dans les liposomes de l'un des surfactants artificiels faisant l'object de la présente invention peut varier entre 1 $mg \cdot ml^{-1}$ et 100 $mg \cdot ml^{-1}$.

L'invention s'étend aussi aux compositions pharmaceutiques ayant comme véhicule les surfactants artificiels décrits ci-dessus, sous une forme adéquate et contenant un ou plusieurs autres composés utilisables en thérapeutique lors du traitement de maladies occasionnées par un déficit en surfactant pulmonaire endogène, tels que enzymes, vitamines, etc...

Les composants des surfactants artificiels de l'invention sont des substances connues et commercialisées. Leurs préparations sont décrites dans la littérature.

La dipalmitoylphosphatidylcholine est notamment commercialisée par SIGMA CHEMICAL COMPANY St. LOUIS, Mo, U.S.A. (pureté 99 %). Elle a un poids moléculaire de 734 et a pour formule chimique:

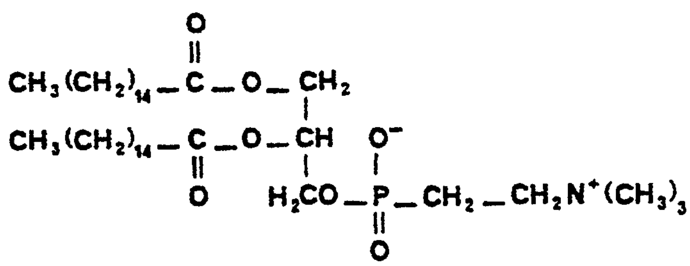

La distéaroylphosphatidylcholine est aussi un composé actuellement commercialisé par SIGMA CHEMICAL COMPANY St LOUIS, Mo, U.S.A. (pureté 99%). Son poids moléculaire est de 790 et sa formule chimique est la suivante:

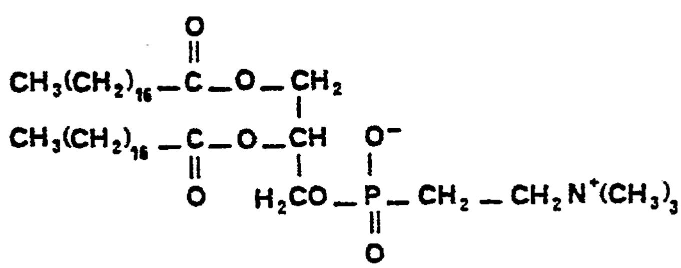

La lécithine de soja est une matière première utilisée depuis longtemps en médecine humaine et spécialement pour la préparation des formes galéniques administrées par voie intraveineuse. Elle est notamment commercialisée sous la marque "Epikuron 200" ® par LUCAS MEYER (Hamburg R.F.A.). Sa composition exacte est connue (Bergenstahl B. et al., Progr. Colloid. & Polymer. Sci. (1983), 68, 48-52). Son poids moléculaire est de 780. La lécithine de soja est d'approvisionnement aisé, de coût faible et ses lots sont de qualité et composition constante.

Les liposomes contenant un des surfactants artificiels de l'invention peuvent être préparés selon le procédé décrit par Bangham (Bangham A.D. Ann. Rev. Biochem., 1972, 41, 735-776). Les constituants du surfactant sont dissous dans un solvant approprié tel que le chloroforme. La solution ainsi obtenue est soumise à une évaporation rotative sous pression réduite à une température comprise de 30-50°C jusqu'à élimination totale du solvant et formation d'un film lipidique fin. Les dernières traces du solvant sont entraînées par un courant d'azote et ensuite le film est repris par une quantité d'eau ou par une solution aqueuse physiologiquement acceptable et soumis à un mouvement rotatoire continu. Les lipides ainsi dispersés dans la phase aqueuse forment spontanément des liposomes.

Pour obtenir des liposomes de taille bien définie et stériles, certaines précautions et étapes supplémentaires sont nécessaires ; les liposomes sont préparés sous des conditions aseptiques et après formation sont soumis à l'action des ultrasons et à une filtration stérilisante.

Les surfactants artificiels selon l'invention ont des propriétés physicochimiques et plus particulièrement des propriétés tensio-actives fort intéressantes et se distinguent des autres surfactants artificiels déjà connus.

En effet, les essais in vitro ont démontré qu'ils répondent a tous les critères de qualité d'un surfactant pulmonaire exogène et qu'ils possèdent des propriétés de surfaces identiques à celles des surfactants naturels. De plus, les essais in vivo ont montré qu'ils sont bien tolérés chez l'animal.

Le surfactant endogène est considéré comme une couche phospholipidique monomoléculaire à l'interface alvéolaire. La description des propriétés caractéristiques d'une telle couche tensioactive in vitro se fait à l'aide d'une courbe pression-aire, montrant la relation entre la pression de surface observée et l'aire occupée sur la surface liquide par les molécules du film. La pression de surface Il, définie comme la diminution de la tension interfaciale produite par la monocouche, est égale à la différence entre la tension de surface du liquide pur et la tension de surface du liquide recouvert du film tensio-actif. Cet abaissement de tension de surface est le critère majeur d'activité d'un surfactant. Les isothermes pression de surface-aire sont réalisées sur une balance à film, dans les conditions proches de la réalité physiologique, au cours de cycles compression-expansion simulant les cycles respiratoires expiration-inspiration. Un bon surfactant exogène doit avoir en fin de compression une pression de surface voisine de 72 $mN \cdot m^{-1}$, ce qui correspond à une tension de surface voisine de zéro.

L'étude des isothermes pression de surface-aire du surfactant pulmonaire du lapin (Beppu O. et al., J. Appl. Physiol. :Respirat. Environ. Exercise Physiol. 1983, 55(2)496-502) et d'un surfactant naturel isolé du liquide amniotique humain (Hallman M. et al., Pediatrics, 1983, 71, N°4, 473-482) a mis en évidence récemment, à part le plateau de collapse final, l'existence d'un plateau intermédiaire. Grâce à ces deux plateaux et donc à la coexistence de deux phases solide et fluide des constituants du surfactant sont obtenues à la fois une haute pression de surface en fin de compression nécessaire pour éviter le collapsus alvéolaire, ainsi qu'une pression non négligeable aux grandes aires qui permet de contribuer à la diminution de l'effort ventilatoire. Cette coexistence des phases est aussi impliquée dans l'amélioration du reétalement du surfactant, nécessaire pour une longue activité des substances tensio-actives.

Les isothermes pression de surface-aire des compositions de surfactant artificiel de l'invention présentent également ce plateau intermédiaire. Ces compositions possèdent alors les caractéristiques des surfactants naturels indiquées ci-dessus. D'autre part, les études in vitro ont démontré que les compositions de l'invention s'adsorbent spontanément à l'interface et provoquent une pression maximale en fin de compression voisine de 72 $mN \cdot m^{-1}$. Cette pression correspond comme il a été indiqué ci-dessus à la pression nécessaire pour éviter le collapsus alvéolaire. De plus les compositions de l'invention sont aussi douées d'une activité très durable. En effet, l'étude des isothermes entre les cycles successifs de compression-expansion a mis en évidence un très faible décalage en aire, ce qui garantit une très longue activité lors de leurs applications in vivo.

Les compositions de surfactant artificiel de l'invention répondent ainsi à tous les critères d'un excellent surfactant exogène et trouvent leurs applications en thérapeutique animale et humaine pour le traitement des maladies occasionnées par un déficit en surfactant pulmonaire endogène.

Les exemples suivants illustrent l'invention.

EXEMPLE 1:

Liposomes de surfactant artificiel constitué d'un mélange de dipalmitoylphosphatidylcholine, de distéaroylphosphatidylcholine et de lécithine de soja (4M:4M:2M).

Dans un ballon rond rodé, préalablement lavé à l'eau distillée stérile apyrogène et séché à 180°C pendant 2 heures, introduire 220 µmoles de dipalmitoylphosphatidylcholine 200 µmoles de distéaroylphosphatidylcholine et 100 µmoles de lécithine de soja. Dissoudre le mélange dans 150 ml de chloroforme, évaporer le solvant à l'aide d'un évaporateur rotatif, sous pression réduite et à une température de 30-50°C. Le film lipidique fin ainsi obtenu est mis sous courant d'azote afin d'éliminer toutes traces de solvant résiduel. Ajouter dans le ballon 76,5 ml d'eau stérile apyrogène. Agiter et laisser sous mouvement rotatoire continu pendant 30 minutes. Les lipides ainsi dispersés dans la phase aqueuse forment spontanément des liposomes.

Transférer les liposomes sous des conditions aseptiques dans une cellule de verre et placer la partie inférieure d'une sonde à ultrasons dans la suspension des liposomes.

Après 20 minutes est obtenue une solution opalescente presque limpide. Filtrer cette solution sur filtre ayant des pores de 0,22 µm de diamètre, puis recueillir et répartir le filtrat sous conditions stériles dans des flacons type "pénicilline".

Les liposomes ainsi obtenus ont une concentration au surfactant artificiel de 5 $mg.ml^{-1}$. Ils doivent être conservés à la température de 4°C et à l'abri de la lumière.

EXEMPLE 2:

Composition pharmaceutique contenant 0.76 $mg \cdot ml^{-1}$ de dl$\alpha$-tocophérol et 20 $mg \cdot ml^{-1}$ de surfactant artificiel constitué d'un mélange de dipalmitoylphosphatidylcholine, de distéaroylphosphatidylcholine et de lécithine de soja (7M:1M:2M).

Dans un ballon rond rodé stérile et sec introduire 280 µmoles de dipalmitoylphosphatidylcholine, 40 µmoles de distéaroylphosphatidylcholine et 80 µmoles de lécithine de soja et 26,5 µmoles de dlα-tocophérol. Dissoudre dans 100 ml de chloroforme. Evaporer le solvant à l'aide d'un évaporateur rotatif sous pression réduite et à une température de 30-50°C, et éliminer ensuite ses traces sous courant d'azote. Ajouter dans le ballon 15 ml d'une solution apyrogène stérile contenant du chlorure de sodium à la concentration de 9‰. Agiter et laisser sous mouvement rotatoire continu pendant 45 minutes et observer la formation des liposomes.

Procéder ensuite à un traitement par les ultrasons et une filtration stérilisante, selon le procédé décrit dans l'exemple 1. Les liposomes stériles ainsi obtenus ont une concentration de 0,76 $mg \cdot ml^{-1}$ en dla-tocophérol et de 20 $mg \cdot ml^{-1}$ en surfactant artificiel.

ETUDE D'INNOCUITE

EXEMPLE 3

Etude d'innocuité chez le rat

L'innocuité des compositions de surfactant artificiel de l'invention a été étudiée chez le rat. La trachée des animaux anesthésiés au nembutal, est perforée au moyen d'une petite aiguille. Un cathéter relié à une seringue de 10 ml contenant le surfactant artificiel est alors introduit par la perforation dans la trachée sur une longueur d'environ 1 cm. La seringue est disposée sur une pompe et l'une ou l'autre des compositions de surfactant décrites dans l'exemple 1 et 2 sont alors perfusées dans la trachée à la dose de 10 $mg \cdot kg^{-1}$ et 60 $mg \cdot mg^{-1}$ avec un débit de 0,95 ml $heure^{-1}$. En fin d'expérimentation, le cathéter est retiré, les plans musculaires et cutanés sont suturés et l'animal reçoit une injection intra-musculaire de 200 000 unités internationales de Pénicilline G.. Les animaux sont par la suite régulièrement pesés et observés jusqu'au jour du sacrifice. 48 heures ou une semaine après l'intervention, les animaux sont sacrifiés par section des carotides. Les poumons sont prélevés et examinés en microscopie optique et électronique. Aucun signe pathologique et modification n'ont été observés.

ETUDE DES PROPRIETES DE SURFACE

EXEMPLE 4:

Etudes des propriétés de surface des compositions de surfactant artificiel

Les propriétés de surface des compositions de surfactant artificiel de l'invention sous forme de liposomes ont été étudiées avec une balance à film type Langmuir. L'étalonnage en pression de surface a été réalisé par simulation d'une pression de surface déterminée à l'aide d'un poids de 0,50008g. Les compositions ont été étudiées sur des sous-phases contenant des sels à concentration physiologique et plus précisément des solutions de chlorure de sodium 0.15 molaire. Les compositions du surfactant artificiel, sous forme de liposomes, sont étalées à l'interface air-eau selon la technique de Verger et Pattus (Chem. Phys. Lipids, 1976, 16, 285-291) et pour l'équilibration, un temps de latence de 15 minutes est respecté avant le début de chaque compression.
Les figures 1, 2 représentent les isothermes des exemples 1 et 2 à 37°C. Sur les courbes, les traits pleins indiquent les compressions des films et les traits en pointillés indiquent les expansions. Les nombres sur les cycles indiquent les numéros d'ordre des cycles.

L'étude de ces courbes mène à des conclusions suivantes:

1/ Les compositions des exemples 1 et 2 sont actives à 37°C.

2/ Elles s'adsorbent spontanément à l'interface et les deux plateaux de collapse apparaissent dès les premiers cycles.

3/ Les films monomoléculaires des compositions de surfactant des exemples 1 et 2 sont capables de générer une pression de surface maximale voisine de 72 $mN \cdot m^{-1}$, avec un premier plateau apparaissant à une pression de surface voisine de 46 $mN \cdot m^{-1}$. Ces deux plateaux qui correspondent d'ailleurs à la coexistence de phases solide et liquide des constituants permettent l'obtention à la fois d'une forte pression de surface en fin de compression nécessaire pour éviter au niveau biologique le collapsus alvéolaire ainsi qu'une pression non négligeable aux grandes aires qui permet de contribuer à la diminution de l'effort ventilatoire.

4/ Les isothermes pression de surface-aire montrent que le plateau de collapse reste important même après plusieurs cycles et seul, un léger déplacement vers les aires les plus faibles apparait entre le deuxième et seizième (exemple 1) ou entre le deuxième et dix-septième (exemple 2) cycle de compression-expansion. Ce phénomène est relié à la grande capacité de réétalement du surfactant, et démontre que la perte des molécules au cours des cycles successifs, due au passage du collapse est minime. Cette propriété est très importante, parce qu'elle garantit l'activité du surfactant pendant une période maximale.

L'étude des propriétés de surface montre que les compositions de l'invention possèdent toutes les caractéristiques d'un bon surfactant exogène.

ETUDE CLINIQUE

EXEMPLE 5:

Etude de l'efficacité immédiate et la tolérance clinique chez le nouveau-né.

L'efficacité immédiate et la tolérance des surfactants artificiels, objet de la présente invention ont été évaluées chez le nouveau-né. Des essais ont été réalisés chez quatre enfants nés très prématurément dont l'espérance de survie était très faible, atteints d'une maladie des membranes hyalines nécessitant un traitement par ventilation artificielle et forte oxygénothérapie (Fi ≧ 50%). Des liposomes de surfactant artificiel, constitué d'un mélange de dipalmitoylphosphatidylcholine, de distearoylphosphatidylcholine et de lecithine de soja (4M:4M:2M) ont été utilisés lors de l'étude. La concentration en surfactant artificiel de liposomes était de 10 $mg.ml^{-1}$. Le surfactant artificiel a été administré entre la 4ème et la 12ème heure de vie, par voie endotrachéale directe. Après avoir incliné l'enfant sur le côté gauche, tête tournée à droite, un fin cathéter a été passé dans la sonde d'intubation et une injection directe de 2 $ml.kg^{-1}$ du produit a été réalisée. Après rebranchement de la ventilation artificielle, la même procédure a été effectuée quelques minutes plus tard, en inclinant l'enfant du côté droit, tête tournée du côté gauche.

La tolérance clinique a toujours été bonne. L'effet immédiat de l'injection a été jugé d'une part par l'augmentation de la pression partielle d'oxygène dans le sang artériel ($\Delta PaO_2$) et d'autre part par la possibilité de diminuer la fraction d'oxygène inspirée ($\Delta FiO_2$). Les résultats de ces essais sont rapportés au tableau I.

Tableau 1

| Enfant | Age gestationnel | Poids | $\Delta PaO_2$ | $\Delta FiO_2$ |
|---|---|---|---|---|
| 1 | 29 sem | 1200 g | + 4,5 KPa (en 5 mn) | –30% (en 2 h) |
| 2 | 29 sem + 3 jours | 1050 g | + 5,9 KPa (en 20 mn) | –20% (en 3 h) |
| 3 | 30 sem | 1300 g | + 4,8 KPa (en 10 mn) | –15% (en 1 h) |
| 4 | 26 sem + 4 jours | 950 g | + 5,4 KPa (en 10 mn) | –35% (en 2 h) |

sem: semaines

**Revendications pour les Etats contractants: BE, CH, DE, FR, GB, IT, LI, LU, NL, SE**

1. Surfactants artificiels pulmonaires caractérisés en ce qu'ils sont constitués par:
1. 10 à 70% en moles de dipalmitoylphosphatidylcholine,
2. 10 à 70% en moles de distéaroylphosphatidylcholine,
3. 10 à 30% en moles de lécithine de soja,
la somme des constituants indiquée sous 1 à 3 devant, dans chaque cas, être égale à 100% en moles.

2. Surfactant artificiel pulmonaire selon la revendication 1 caractérisé en ce qu'il est constitué de 40% en moles de dipalmitoylphosphatidylcholine, 40% en moles de distéaroylphosphatidylcholine et 20% en moles de lécithine de soja.

3. Compositions pharmaceutiques contenant seulement un surfactant artificiel selon l'une des revendications 1 à 2, sous une forme adéquate, utilisables pour remplacer le surfactant endogène pulmonaire défaillant.

4. Compositions pharmaceutiques selon la revendication 3, sous forme de liposomes éventuellement mis en suspension dans l'eau ou dans une solution aqueuse physiologiquement acceptable.

5. Compositions pharmaceutiques contenant un ou plusieurs composés utilisables en thérapeutique et renfermant à titre de véhicule un surfactant artificiel pulmonaire selon l'une des revendications 1 à 2.

**Revendications pour l'Etat contractant: AT**

1. Procédé de préparation des surfactants artificiels caractérisés en ce qu'ils sont constitués par:
1. 10 à 70% en moles de dipalmitoylphosphatidylcholine,
2. 10 à 70% en moles de distéaroylphosphatidylcholine,
3. 10 à 30% en moles de lécithine de soja,
la somme des constituants indiquée sous 1 à 3 devant, dans chaque cas, être égale à 100% en moles.

2. Procédé de préparation de surfactant artificiel selon la revendication 1 caractérisé en ce qu'il est constitué de 40% en moles de dipalmitoylphosphatidylcholine, 40% en moles de distéaroylphosphatidylcholine et 20% en moles de lécithine de soja.

3. Utilisation d'un surfactant artificiel pulmonaire selon l'une des revendications 1 à 2, sous une forme adéquate, pour préparer des compositions utilisables pour remplacer le surfactant endogène pulmonaire défaillant chez les mamifères.

4. Méthode de préparation des compositions selon la revendication 3, sous forme de liposomes éventuellement mis en suspension dans l'eau ou dans une solution aqueuse physiologiquement acceptable.

5. Utilisation d'un surfactant artificiel pulmonaire selon l'une des revendications 1 à 2 à titre de véhicule, pour préparer de compositions contenant un ou plusieurs composés utilisables en thérapeutique chez les mammifères.

**Patentansprüche für die Vertragsstaaten: BE, CH, DE, FR, GB, IT, LI, LU, NL, SE**

1. Synthetische oberflächenaktive Mittel für die Lunge, dadurch gekennzeichnet, daß sie aus
1) 10 bis 70 Mol.-% Dipalmitoylphosphatidylcholin,
2) 10 bis 70 Mol.-% Distearoylphosphatidylcholin und
3) 10 bis 30 Mol.-% Soja-Lecithin
gebildet sind, wobei die Summe der Bestandteile 1) bis 3) in jedem Fall 100 Mol.-% entspricht.

2. Synthetisches oberflächenaktives Mittel für die Lunge nach Anspruch 1, dadurch gekennzeichnet, daß es aus 40 Mol.-% Dipalmitoylphosphatidylcholin, 40 Mol.-% Distearylphosphatidylcholin und 20 Mol.-% Soja-Lecithin gebildet ist.

3. Pharmazeutische Zubereitungen enthaltend lediglich ein synthetisches oberflächenaktives Mittel nach einem der Ansprüche 1 bis 3 in einer geeigneten Form, die den Ersatz von fehlendem endogenem oberflächenaktiven Mittel der Lunge ermöglicht.

4. Pharmazeutische Zubereitungen nach Anspruch 2, in Form von Liposomen, die gegebenenfalls in Wasser oder in einer physiologisch annehmbaren wässrigen Lösung suspendiert sind.

5. Pharmazeutische Zubereitungen enthaltend eine oder mehrere in der Therapie verwendbare Verbindungen und als Träger ein synthetisches oberflächenaktives Mittel für die Lunge nach einem der Ansprüche 1 bis 2.

**Patentansprüche für den Vertragsstaat: AT**

1. Verfahren zur Herstellung von synthetischen oberflächenaktiven Mitteln, dadurch gekennzeichnet, daß sie aus
1) 10 bis 70 Mol.-% Dipalmitoylphosphatidylcholin,
2) 10 bis 70 Mol.-% Distearoylphosphatidylcholin und
3) 10 bis 30 Mol.-% Soja-Lecithin
gebildet werden, wobei die Summe der oben angegebenen Bestandteile 1) bis 3) in jedem Fall 100 Mol.-% entspricht.

2. Verfahren zur Herstellung des synthetischen oberflächenaktiven Mittels nach Anspruch 1, dadurch gekennzeichnet, daß es aus 40 Mol.-% Dipalmitoylphosphatidylcholin, 40 Mol.-% Distearylphosphatidylcholin und 20 Mol.-% Soja-Lecithin gebildet wird.

3. Verwendung eines synthetischen oberflächenaktiven Mittels für die Lunge nach einem der Ansprüche 1 bis 2 in geeigneter Form zur Herstellung von Präparaten, die zum Ersatz des bei Säugern fehlenden endogenen oberflächenaktiven Mittels der Lunge verwendet werden können.

4. Verfahren zur Herstellung von Präparaten nach Anspruch 3, in Form von Liposomen, die gegebenenfalls in Wasser oder in einer physiologisch annehmbaren wässrigen Lösung suspendiert sind.

5. Verwendung eines oberflächenaktiven Mittels für die Lunge nach einem der Ansprüche 1 bis 2 als Träger zur Herstellung von Zubereitungen, die eine oder mehrere für die Therapie von Säugern verwendbare Verbindungen enthalten.

**Claims for the contracting states: BE, CH, DE, FR, GB, IT, LI, LU, NL, SE**

1. Artificial pulmonary surfactants, characterised in that they are composed of
1) from 10 to 70 mol % of dipalmitoylphosphatidylcholine,
2) from 10 to 70 mol % of distearoylphosphatidylcholine,
3) from 10 to 30 mol % of soya lecithin,
wherein the sum of the constituents indicated under 1 to 3 must, in each case, be equal to 100 mol %.

2. Artificial pulmonary surfactant according to claim 1, characterised in that it is composed of 40 mol % of dipalmitoylphosphatidylcholine, 40 mol % of distearoylphosphatidylcholine and 20 mol % of soya lecithin.

3. Pharmaceutical compositions containing only one artificial surfactant according to one of claims 1 and 2, in a suitable form, which pharmaceutical compositions can be used for replacing deficient endogenous pulmonary surfactant.

4. Pharmaceutical compositions according to claim 3, in the form of liposomes optionally suspended in water or in a physiologically acceptable aqueous solution.

5. Pharmaceutical compositions containing one or more compounds which can be used therapeutically and contain as a carrier an artificial pulmonary surfactant according to one of claims 1 and 2.

**Claims for the contracting state: AT**

1. Process for the preparation of artificial surfactants, characterised in that they are composed of
1) from 10 to 70 mol % of dipalmitoylphosphatidylcholine,
2) from 10 to 70 mol % of distearoylphosphatidylcholine,
3) from 10 to 30 mol % of soya lecithin,
wherein the sum of the constituents indicated under 1 to 3 must, in each case, be equal to 100 mol %.

2. Process for the preparation of an artifical surfactant according to claim 1, characterised in that it is composed of 40 mol % of dipalmitoylphosphatidylcholine, 40 mol % of distearoylphosphatidylcholine and 20 mol % of soya lecithin.

3. Use of an artificial pulmonary surfactant according to one of claims 1 and 2, in a suitable form, for the preparation of compositions that can be used for replacing deficient endogenous pulmonary surfactant in mammals.

4. Method or preparing compositions according to claim 3, in the form of liposomes optionally suspended in water or in a physiologically acceptable aqueous solution.

5. Use of an artificial pulmonary surfactant according to one of claims 1 and 2 as a carrier for the preparation of compositions containing one or more compounds which can be used therapeutically in mammals.

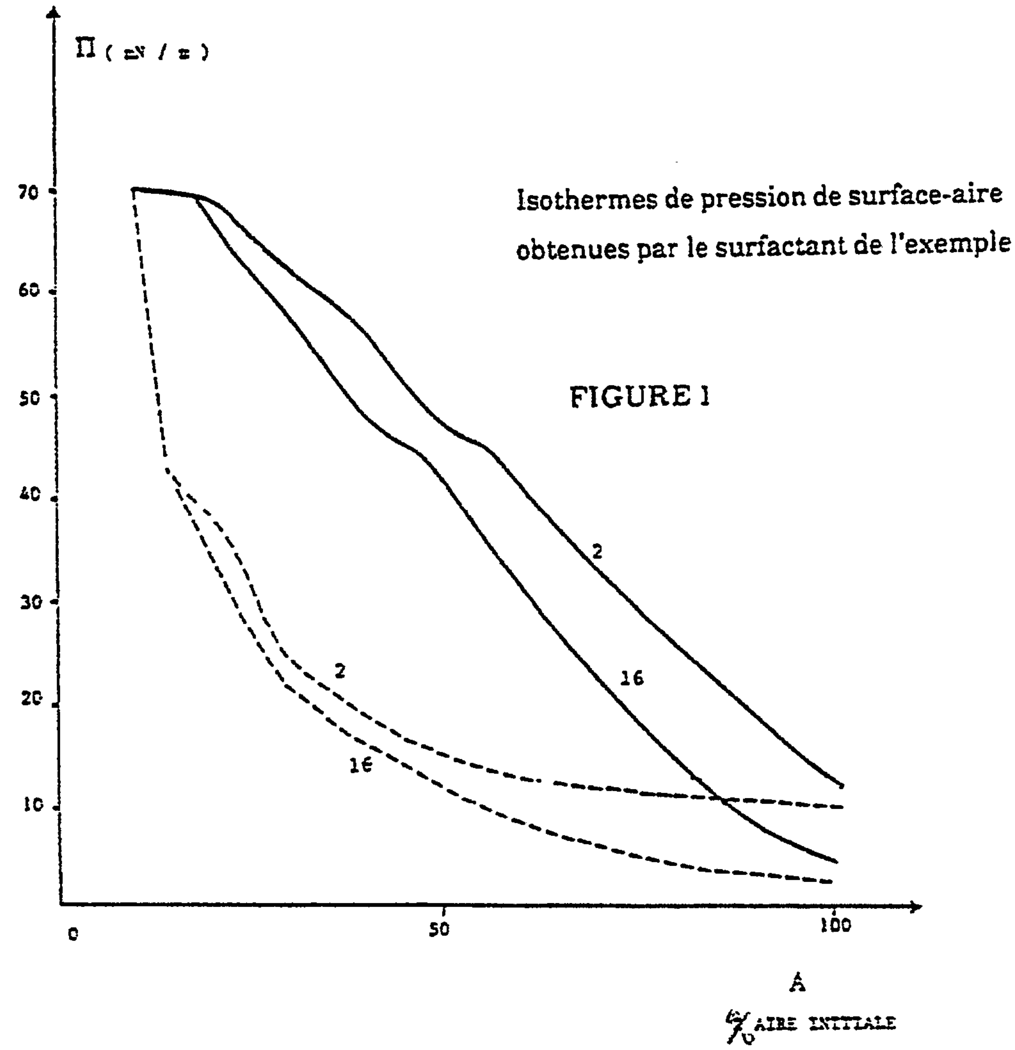


Isothermes de pression de surface-aire obtenues par le surfactant de l'exemple 1

FIGURE 1

Isothermes de pression de surface-aire obtenues par le surfactant de l'exemple 2

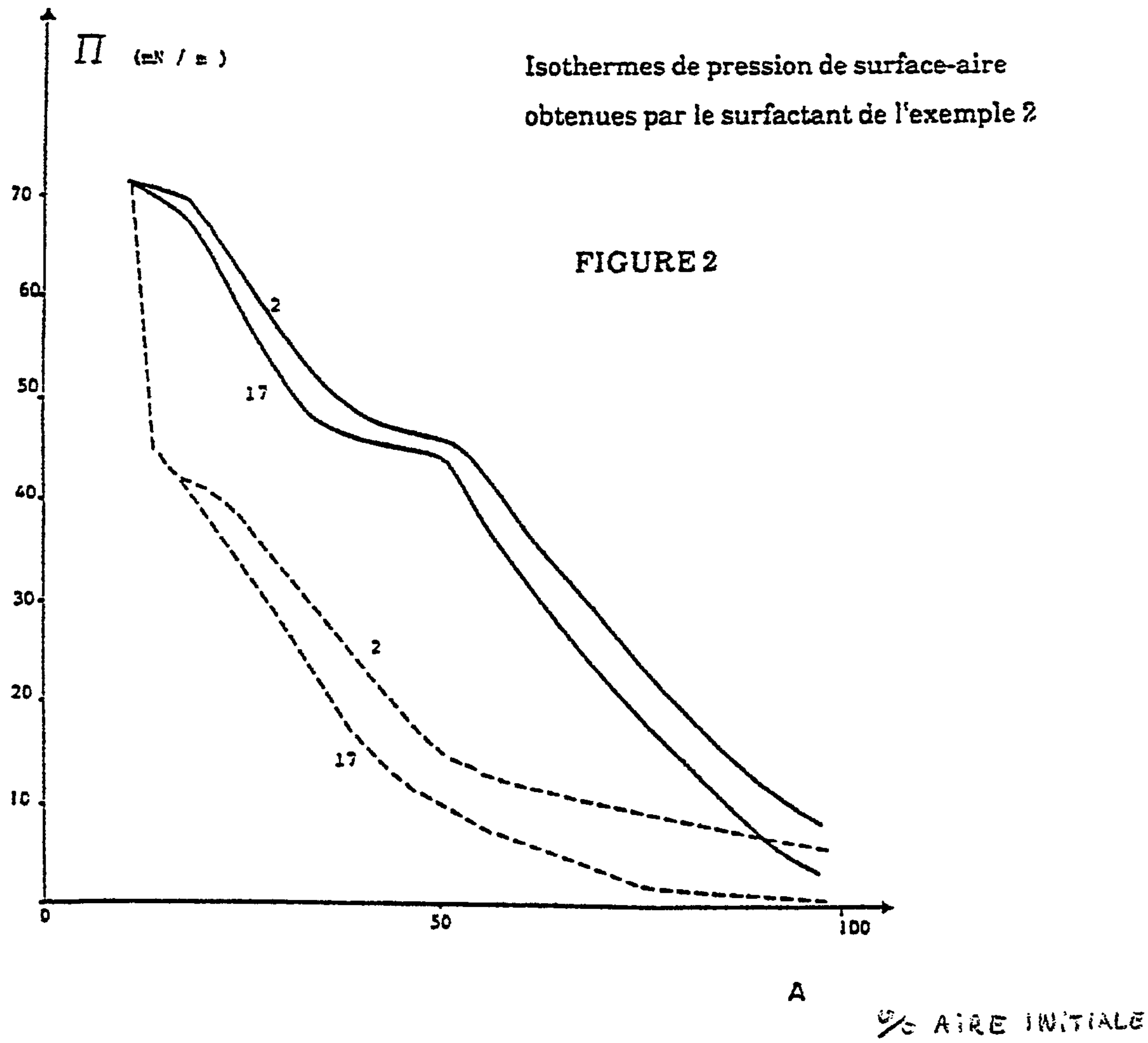


FIGURE 2